# FASCICULE DE BREVET EUROPEEN

(11) **EP 0 593 641 B1**
(45) Date de publication et mention de la délivrance du brevet: **27.12.1995**
(21) Numéro de dépôt: 92915477.1
(22) Date de dépôt: 08.07.1992
(51) Int. Cl.: C07D 305/14

(54) **PROCEDE DE PREPARATION DE DERIVES DE LA BACCATINE III ET DE LA DESACETYL-10 BACCATINE III**
Verfahren zur Herstellung von Bacchatin III und von Desacetyl-10-Bacchatin III Derivate
PROCESS FOR THE PREPARATION OF BACCATIN III AND 10-DEACETYL BACCATIN III DERIVATIVES

(30) Priorité: 10.07.1991 FR 9108673
(43) Date de publication de la demande: 27.04.1994
(73) Titulaire: RHONE-POULENC RORER S.A., 92160 Antony (FR)
(72) Inventeur: HITTINGER, Augustin, F-91430 Igny (FR)
(74) Mandataire: Pilard, Jacques
(86) Numéro de dépôt international: FR9200649
(87) Numéro de publication internationale: WO9301179

(56) Documents cités:
- EP-A- 253 738
- EP-A- 0 336 841
- JOURNAL OF THE AMERICAN CHEMICAL SOCIETY. vol. 110, 1988, GASTON, PA US pages 5917 - 5918; J-N. DENIS ET AL: 'A HIGHLY EFFICIENT, PRACTICAL APPROACH TO NATURAL TAXOL'
- TETRAHEDRON vol. 42, no. 16, 1986, OXFORD pages 4451 - 4460; F. GUERITTE-VOEGELEIN ET AL.: 'CHEMICAL STUDIES OF 10-DEACETYL BACCATIN III. HEMISYNTHESIS OF TAXOL DERIVATIVES'
- JOURNAL OF ORGANIC CHEMISTRY. vol. 51, 1986, EASTON US pages 3239 - 3242; N. F. NAGRI ET AL.: 'MODIFIED TAXOLS III. PREPARATION AND ACYLATION OF BACCATIN III.'

## Description

La présente invention concerne un procédé de préparation de dérivés de la baccatine III et de la désacétyl-10 baccatine III de formule générale : 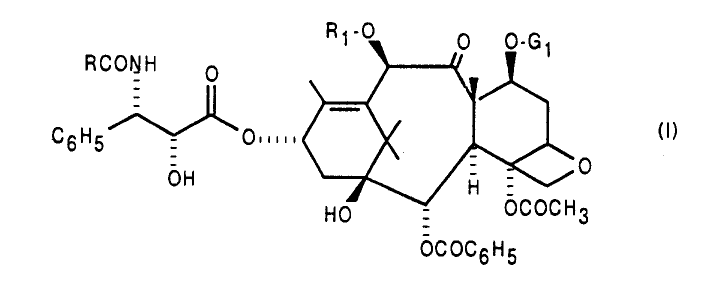
dans laquelle R représente un radical t.butoxy ou phényle, R₁ représente un radical acétyle ou un groupement protecteur de la fonction hydroxy sous forme d'un éther, d'un ester ou d'un carbonate et G₁ représente un groupement protecteur de la fonction hydroxy sous forme d'un éther, d'un ester ou d'un carbonate sous forme 2'R,3'S ou 2'S,3'S.

Plus particulièrement la présente invention concerne un procédé de préparation d'un produit de formule générale (I) dans laquelle R représente un radical t.butoxy ou phényle, R₁ représente un radical acétyle ou trichloro-2,2,2 éthoxycarbonyle et G₁ représente un radical trichloro-2,2,2 éthoxycarbonyle sous forme 2'R,3'S ou 2'S,3'S.

Les produits de formule générale (I) sont particulièrement utiles dans la synthèse des produits de formule générale : 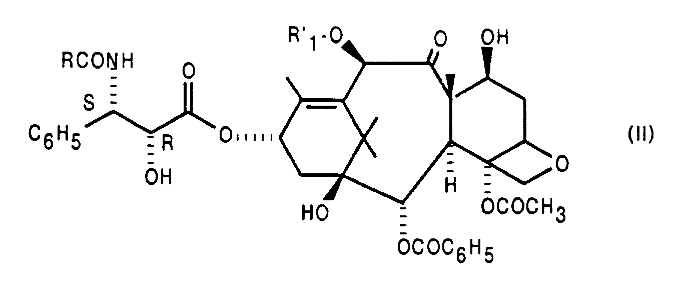
dans laquelle R est défini comme précédemment et R'₁ représente un atome d'hydrogène ou un radical acétyle.

Les produits de formule générale (II) dans laquelle R représente un radical phényle correspondent au taxol et au désacétyl-10 taxol et les produits de formule générale (II) dans laquelle R représente un radical t.butoxy correspondent à ceux qui font l'objet du brevet européen EP 0 253 738.

Les produits de formule générale (II) manifestent des activités antitumorales et antileucémiques particulièrement intéressantes.

Les produits de formule générale (I) dans laquelle R représente un radical t.butoxy peuvent être obtenus dans les conditions décrites dans le brevet européen EP 0 253 738 après séparation des produits provenant de l'hydroxyamination d'un produit de formule générale : 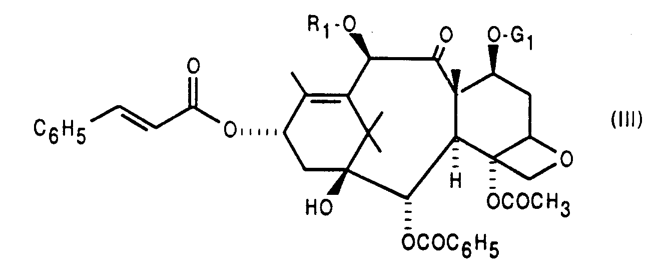
dans laquelle R₁ représente un radical acétyle ou trichloro-2,2,2 éthoxycarbonyle et G₁ représente un radical trichloro-2,2,2 éthoxycarbonyle.

Les produits de formule générale (I) dans laquelle R représente un radical t.butoxy sont transformés en produits de formule générale (II) correspondant par élimination des groupements protecteurs représentés par R₁ et G₁. Plus particulièrement, lorsque R₁ et G₁ représentent un radical trichloro-2,2,2 éthoxycarbonyle, ce remplacement est effectué par action du zinc en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone en présence de zinc dans les conditions décrites dans le brevet européen EP 0 253 738.

D'après les brevets américains US 4 924 011 et US 4 924 012 il est connu de préparer les produits de formule générale (II) par condensation d'un dérivé de la β-phénylisosérine de formule générale : 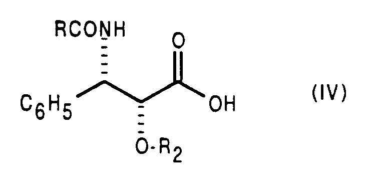
dans laquelle R est défini comme précédemment et R₂ représente un groupement protecteur de la fonction hydroxy choisi notamment parmi les radicaux méthoxyméthyle, éthoxy-1 éthyle, benzyloxyméthyle, (β-triméthylsilyléthoxy)méthyle, tétrahydropyrannyle ou trichloro-2,2,2 éthoxycarbonyle, sur un dérivé de la baccatine III ou de la désacétyl-10 baccatine III de formule générale : 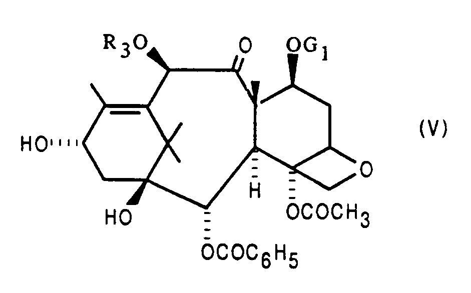
dans laquelle R₃ représente un radical acétyle ou un groupement protecteur de la fonction hydroxy choisi parmi les radicaux trichloro-2,2,2 éthoxycarbonyle ou trialkylsilyle dont chaque partie alcoyle contient 1 à 3 atomes de carbone et G₁ représente un groupement protecteur de la fonction hydroxy choisi parmi les radicaux trichloro-2,2,2 éthoxycarbonyle ou trialkylsilyle dont chaque partie alkyle contient 1 à 3 atomes de carbone, suivie du remplacement des groupements protecteurs des fonctions hydroxy par des atomes d'hydrogène.

Selon la nature des groupements protecteurs, ce remplacement peut être effectué au moyen de zinc en présence d'acide acétique ou d'un acide minéral ou organique tel que l'acide chlorhydrique ou l'acide acétique en solution dans un alcool aliphatique contenant 1 à 3 atomes de carbone en présence de zinc lorsque les groupements protecteurs représentent au moins un radical trichloro-2,2,2 éthoxycarbonyle, soit au moyen d'un acide tel que l'acide chlorhydrique dans un alcool aliphatique contenant 1 à 3 atomes de carbone à une température voisine de 0°C lorsque les groupements protecteurs représentent au moins un radical trialkylsilyle.

Les produits de formule générale (II) obtenus par la mise en oeuvre de ces procédés antérieurement connus doivent être purifiés, généralement par chromatographie ou par cristallisation, afin, plus particulièrement, de séparer les formes épimères 2'R,3'S et 2'S,3'S. Cette Séparation est industriellement de mise en oeuvre délicate du fait de la cytotoxicité des produits de formule générale (II).

Il a maintenant été trouvé que les produits de formule générale (II) pratiquement purs peuvent être obtenus en purifiant préalablement un produit de formule générale (I) puis en éliminant les groupements protecteurs représentés par R₁ et G₁ des produits de formule générale (I) purifiés dans des conditions non épimérisantes.

La présente invention concerne un procédé de préparation d'un produit de formule générale (I) à partir d'un dérivé du taxane de formule générale : 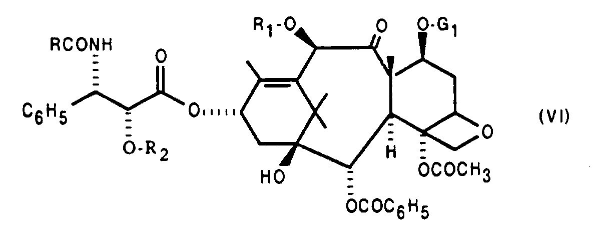
dans laquelle R₁ et G₁ sont définis comme précédemment et R₂ représente un groupement protecteur de la fonction hydroxy choisi parmi les radicaux méthoxyméthyle, éthoxy-1 éthyle, benzyloxyméthyle, (β-triméthylsilyléthoxy)méthyle ou tétrahydropyrranyle, par traitement en milieu acide à une température comprise entre 0 et 30°C. Généralement, on utilise une solution alcoolique d'un acide minéral fort. De préférence on utilise une solution éthanolique d'acide chlorhydrique.

Le produit de formule générale (II) est obtenu en traitant le produit de formule générale (I) dans laquelle R₁ représente un radical acétyle ou trichloro-2,2,2 éthoxycarbonyle et G₁ représente un radical trichloro-2,2,2 éthoxycarbonyle sous forme 2'R,3'S ou 2'S,3'S purifié, généralement par chromatographie ou cristallisation, par le zinc dans l'acide acétique en milieu alcoolique à une température comprise entre 20°C et la température de reflux du mélange réactionnel. De préférence, on opère à une température voisine de 65°C.

Le produit de formule générale (VI) peut être obtenu par action d'un acide de formule générale : 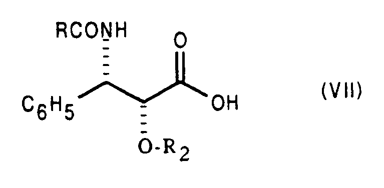
sur un dérivé de la baccatine III ou de la désacétyl-10 baccatine III de formule générale : 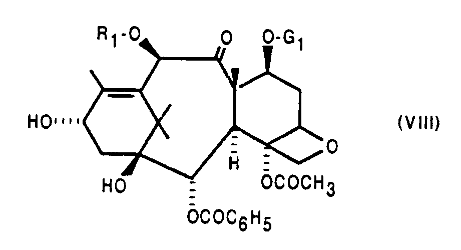
dans laquelle R₁ et G₁ sont définis comme précédemment dans les conditions décrites dans les brevets américains US 4 924 011 et US 4 924 012.

Les produits de formule générale (I) étant dénués de cytotoxicité, leur purification par chromatographie ou cristallisation ne nécessite pas de mesures particulières de sécurité et d'hygiène industrielle.

Les exemples suivants, donnés à titre non limitatif, montrent comment l'invention peut être mise en pratique.

### EXEMPLE 1

Dans un ballon de 500 cm3 muni d'une agitation et d'une ampoule de coulée, on introduit, sous atmosphère d'argon, 23 g de t.butoxycarbonylamino-3 (éthoxy-1 éthoxy)-2 phényl-3 propionate-(2R,3S) d'acétoxy-4 benzoyloxy-2α époxy-5β,20 hydroxy-1β oxo-9 bis-(trichloro-2,2,2 éthoxy) carbonyloxy-7β,10β taxène-11 yl-13α, 200 cm3 de méthanol et 18,7 cm3 d'éthanol chlorhydrique 1N. On agite pendant 30 minutes à une température voisine de 20°C. Après concentration du mélange réactionnel sous pression réduite à une température inférieure à 25°C jusqu'à l'obtention d'un résidu non totalement sec (40 g), on reprend par 200 cm3 de chlorure de méthylène puis on lave par 100 cm3 d'une solution saturée de chlorure de sodium. Après séchage sur sulfate de sodium, filtration et concentration à sec sous pression réduite (25 mm de mercure ; 3,3 kPa, puis 1 mm de mercure ; 0,13 kPa) à 25°C, on obtient une huile incolore (25 g) qui est purifiée par chromatographie sur 610 g de silice (40-63 »m) en éluant avec un mélange cyclohexane-acétate d'éthyle (70-30 en volumes). On obtient ainsi 14,2 g de t.butoxycarbonylamino-3 hydroxy-2 phényl-3 propionate(2R,3S) d'acétoxy-4 benzoyloxy-2α époxy-5β,20 hydroxy-1 oxo-9 bis- (trichloro-2,2,2 éthoxycarbonyloxy-7β,10β taxène-11 yl-13α.

Rf = 0,29 [cyclohexane-acétate d'éthyle (70-30 en volumes).

### EXEMPLE 2

Dans un ballon tricol muni d'une agitation, on introduit sous atmopshère d'argon, l'ester obtenu à l'exemple 1 (11,9 g) puis 200 cm3 d'un mélange méthanolacide acétique (1-1 en volumes) et enfin 6 g de zinc en poudre. On chauffe le mélange pendant 2 heures à 60°C puis on refroidit à 25°C. Après filtration, le principité est lavé par 4 fois 20 cm3 d'un mélange méthanol-acétate d'éthyle (1-1 en volumes). Le filtrat et les lavages sont réunis puis concentrés à sec. On reprend par 200 cm3 de toluène puis concentré à sec sous pression réduite (25 mm de mercure ; 3,3 kPa à 35°C puis 1 mm de mercure : 0,13 kPa à 25°C). Le résidu obtenu est repris par 200 cm3 d'acétate d'éthyle. La solution est lavée par 50 cm3 d'une solution saturée de bicarbonate de sodium puis par 100 cm3 d'eau distillée. La phase organique est séchée sur sulfate de magnésium. Après filtration et concentration à sec sous pression réduite (25 mm de mercure ; 3,3 kPa à 35°C puis 1 mm de mercure ; 0,13 kPa à 25°C), on obtient 10 g de t.butoxycarbonylamino-3 hydroxy-2 phényl-3 propionate-(2R,3S) d'acétoxy-4 benzoyloxy-2α époxy-5β,20 trihydroxy-1,7β,10β oxo-9 taxène-11 yl-13α sous forme d'une poudre blanche qui est purifiée par chromatographie sur silice.

Le produit ainsi obtenu a une pureté supérieure à 99,5%.

### EXEMPLE 3

A une solution de 20,3 g (16,55 mmoles) de t.butoxycarbonylamino-3 (éthoxy-1 éthoxy)-2 phényl-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yl-13α dans 180 cm3 d'éthanol absolu, on ajoute en 10 minutes, à une température voisine de 20°C, 16,6 cm3 d'une solution éthanolique 1N d'acide chlorhydrique. Après 30 minutes, le mélange réactionnel est concentré aux 4/5 de son volume. La résidu huileux obtenu est ajouté lentement à 200 cm3 d'eau fortement agitée contenant une quantité suffisante d'hydrogénocarbonate de sodium pour neutraliser l'excès d'acide chlorhydrique. Il se forme un précipité jaune clair. Après filtration, lavage avec de l'eau permuttée et séchage sous pression réduite, le produit brut obtenu (25 g) est purifié sur une colonne de silice en éluant avec un mélange cyclohexane-acétate d'éthyle (70-30 en volumes). On obtient ainsi 14,5 g de t.butoxycarbonylamino-3 hydroxy-2 phényl-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yl-13α sous forme d'un solide blanc fondant à 185°C et dont le pouvoir rotatoire est [α]_{D} = -32,4° (c = 0,974 ; méthanol).

### EXEMPLE 4

A une solution de 5 g (4,33 mmoles) de l'ester obtenu à l'exemple 3 dans 90 cm3 d'un mélange acide acétique-méthanol (1-1 en volumes) chauffée à 50°C sous atmosphère inerte, on ajoute 2,67 g (40,8 mmoles) de poudre de zinc activé. Le mélange réactionnel est agité pendant 1 heure à 50°C. Après refroidissement, le mélange réactionnel est filtré et le solide est lavé avec 10 cm3 de méthanol. Les filtrats réunis sont concentrés à sec sous pression réduite à 30°C. Le résidu obtenu est repris par 50 cm3 de toluène puis on concentre à nouveau à sec. La meringue blanche obtenue est solubilisée dans 100 cm3 d'acétate d'éthyle. La solution obtenue est lavée avec 25 cm3 d'une solution aqueuse d'hydrogénocarbonate de sodium 1M puis avec 3 fois 50 cm3 d'eau. Après séchage sur sulfate de sodium, filtration et concentration à sec, on obtient 3,34 g d'un solide blanc. Après recristallisation dans un mélange éthanol-eau, on obtient 2,7 g de t.butoxycarbonylamino-3 hydroxy-2 phényl-3 propionate-(2R,3S) d'acétoxy-4 benzoyloxy-2α époxy-5β,20 trihydroxy-1,7β,10β oxo-9 taxène-11 yl-13α sous forme d'un solide blanc fondant à 196°C.

## Revendications

1. Procédé de préparation d'un dérivé de la baccatine III ou de la désacétyl-10 baccatine III de formule générale : 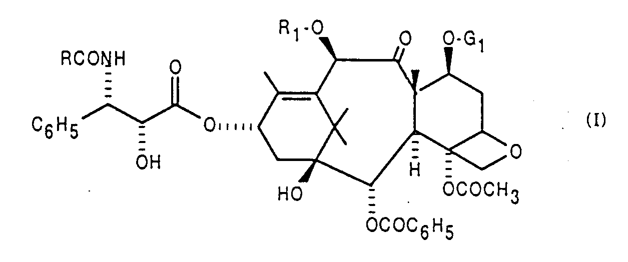 dans laquelle R représente un radical t.butoxy ou phényle, R₁ représente un radical acétyle ou un groupement protecteur de la fonction hydroxy sous forme d'un éther, d'un ester où d'un carbonate et G₁ représente un groupement protecteur de la fonction hydroxy sous forme d'un éther, d'un ester ou d'un carbonate sous forme 2'R,3'S ou 2'S,3'S, caractérisé en ce que l'on traite en milieu acide au moyen d'une solution alcoolique d'un acide fort un dérivé du taxane de formule générale : 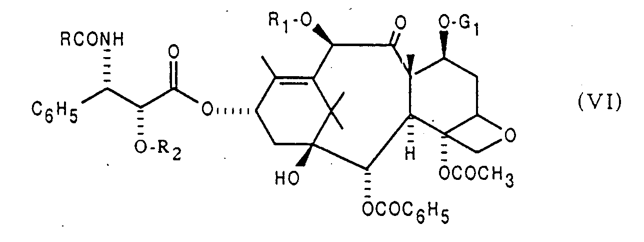 dans laquelle R₁ et G₁ sont définis comme ci-dessus et R₂ représente un groupement protecteur de la fonction hydroxy choisi parmi les radicaux méthoxyméthyle, éthoxy-1 éthyle, benzyloxyméthyle, (β-triméthylsilyléthoxy) méthyle ou tétrahydropyrannyle.

2. Procédé de préparation selon la revendication 1 d'un dérivé de la baccatine III ou de la désacétyl-10 baccatine III de formule générale (I) dans laquelle R représente un radical t.butoxy ou phényle et R₁ représente un radical acétyle ou trichloro-2,2,2 éthoxycarbonyle et G₁ représente un radical trichloro-2,2,2 éthoxycarbonyle sous forme 2'R,3'S ou 2'S,3'S

3. Procédé selon la revendication 1 caractérisé en ce que l'on utilise une solution éthanolique d'acide chlorhydrique.

## Claims

1. Process for the preparation of a derivative of baccatin III or of 10-deacetylbaccatin III, of general formula: 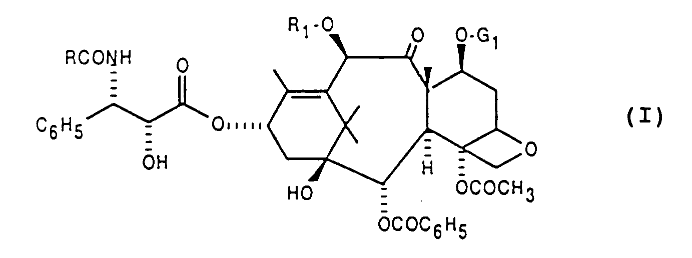 in which R represents a tert-butoxy or phenyl radical, R₁ represents an acetyl radical or a protecting group of the hydroxyl functional group in the form of an ether, ester or carbonate and G₁ represents a protecting group of the hydroxyl functional group in the form of an ether, ester or carbonate, in the 2'R,3'S or 2'S,3'S form, characterised in that a taxane derivative of general formula: 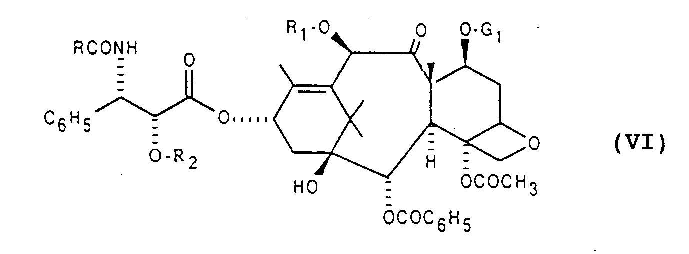 in which R₁ and G₁ are defined as above and R₂ represents a protecting group of the hydroxyl functional group chosen from the methoxymethyl, 1-ethoxyethyl, benzyloxymethyl, (β-trimethylsilylethoxy)methyl or tetrahydropyranyl radicals, is treated in acid medium using an alcoholic solution of a strong acid.

2. Process for the preparation according to claim 1 of a derivative of baccatin III or of 10-deacetylbaccatin III of general formula (I) in which R represents a tert-butoxy or phenyl radical and R₁ represents an acetyl or 2,2,2-trichloroethoxycarbonyl radical and G₁ represents a 2,2,2-trichloroethoxycarbonyl radical, in the 2'R,3'S or 2'S,3'S form.

3. Process according to claim 1, characterised in that an ethanolic solution of hydrochloric acid is used.

## Patentansprüche

1. Verfahren zur Herstellung eines Derivats von Bacchatin III oder 10-Desacetylbacchatin III der allgemeinen Formel: 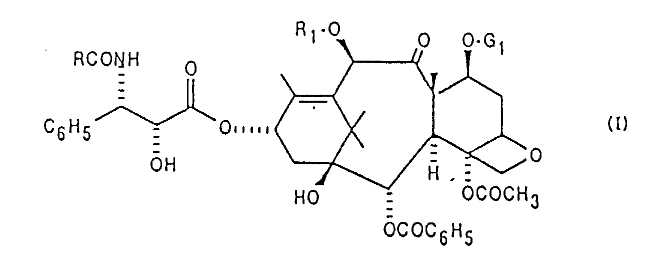 in der R einen tert.-Butoxy- oder Phenylrest darstellt, R₁ einen Acetylrest oder eine Schutzgruppe der Hydroxy-Funktion in Form eines Ethers, eines Esters oder eines Carbonats darstellt und G₁ eine Schutzgruppe der Hydroxy-Funktion in Form eines Ethers, eines Esters oder eines Carbonats darstellt, in der Form 2'R,3'S oder 2'S,3'S, dadurch gekennzeichnet, daß man in saurem Medium mittels einer alkoholischen Lösung einer starken Säure ein Taxan-Derivat behandelt, das die allgemeine Formel: 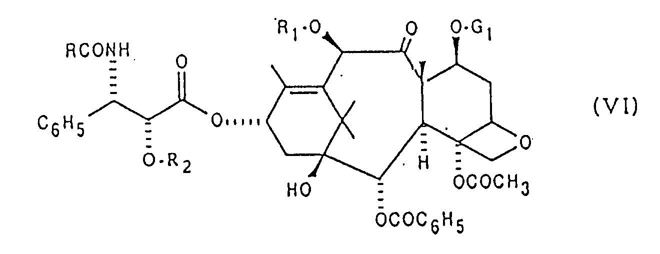 aufweist, in der R₁ und G₁ wie vorstehend definiert sind und R₂ eine Schutzgruppe der Hydroxy-Funktion darstellt, die aus dem Methoxymethyl-, 1-Ethoxyethyl-, Benzyloxymethyl-, (β-Trimethylsilylethoxy)methyl- oder Tetrahydropyranyl-Rest ausgewählt ist.

2. Verfahren zur Herstellung eines Derivats von Bacchatin III oder von 10-Desacetylbacchatin III der allgemeinen Formel (I) nach Anspruch 1, in der R einen tert.-Butoxy- oder Phenylrest darstellt und R₁ einen Acetyl- oder 2,2,2-Trichlorethoxycarbonyl-Rest darstellt und G₁ einen 2,2,2-Trichlorethoxycarbonyl-Rest darstellt, in der Form 2'R,3'S oder 2'S,3'S.

3. Verfahren nach Anspruch 1, dadurch gekennzeichnet, daß man eine ethanolische Lösung von Chlorwasserstoffsäure verwendet.
